# EUROPEAN PATENT APPLICATION

(11) **EP 0 995 458 A2**
(43) Date of publication of application: **26.04.2000**
(21) Application number: 99120041.1
(22) Date of filing: 18.10.1999
(51) Int. Cl.: A61M 21/00

(54) **Method for communicating subliminal visual messages**

(30) Priority: 21.10.1998 IT BO980595
(71) Applicant: Silingardi, Vitaliano, 40137 Bologna (IT)
(72) Inventor: Silingardi, Vitaliano, 40137 Bologna (IT)
(74) Representative: Dall'Olio, Giancarlo

(57) **Abstract**

According to the claimed method, before the step in which the subliminal message is presented, a previous step is performed, in which a predetermined image, or a sequence of images, is displayed for a predetermined time. The aim of this previous step is that of drawing the attention of the observer to the message that is displayed immediately after. The invention includes various embodiments for putting this previous step into practice.

## Description

After the World War II, subliminal communication has been raising a certain interest.

This interest has derived from the fact that if subliminal messages had been effectively communicated, they could have been used for psychological therapy programs which were intended to aid in treatment of mental or emotional disorders.

These emotional disorders include, for example, anxiety. stress, sleeplessness, neurosis, slight depression.

Subliminal messages can be also useful for treatment of psychosomatic diseases. In addition, applications of subliminal messages include programs for psychological dependence control (smoking or food dependence and so on). An interesting application of subliminal messages could be directed to increase the psychic tension and to strengthen the deep will to recover in people with serious diseases: this could bring, at least, to an improvement of the quality of life.

Disregarding reports or imaginary applications, which belong rather to a fantasy world, in the following, we will carefully examine what has been done worth taking into consideration.

During the last forty years, many academic researchers have conducted researches concerning visual subliminal messages. These researchers have used an optical-mechanical instrument known as tachistoscope, including an arrangement of mirrors, lenses and an electromechanical shutter.

With this instrument, an image can be replaced by a message for some milliseconds (i.e. three or four). The message may include images or, more frequently, words.

Typically, a human subject is shown first a blank image on a card and, at some point in time, the shutter is activated to expose to the subject another card carrying the message. The exposure is very brief and lasts for a controlled period of time, before a blank image is restored.

The blank image is very bright to erase the message image still persisting on the retina of the subject.

The findings of the researches conducted both in Europe and in the United States are uncertain or negative.

Researches have been conducted about communicating subliminal messages by means of a computer and exploiting this same principle. Some of the experiments have been very sophisticated and included the possibility of embedding the subliminal messages in television programmes or in movies at the cinemas.

However, also the best conducted and more complete researches do not introduce any novel feature in the message characteristics with respect to the messages shown with the tachistoscope. As a matter of fact, the results are generally less satisfactory.

We do not take into consideration also those investigations and researches which concern methods and devices which present the subliminal message confused with a background image, due to graphics or low contrast. In fact, most valuable scientists who have conducted researches with these methods, have come to the conclusion that they do not give a solid base for activation of subliminal stimuli.

As a consequence of what has been said heretofore, the attention of the inventor has been focused on the discovery of possible errors occurring in the prior art methodology. According to the prior art methodology, the image containing the subliminal message, generally including one or more words, is presented for some milliseconds in place of a previous image, which can be casual (this is the typical case of a subliminal message inserted in a movie or in a television programme) or a white image as in the case of the message written on a white background as in most of the experiments conducted with the tachistoscope in psychological laboratories.

According to the most accurate methods, the message is replaced after a few milliseconds with an eraser image, which is a monochromatic image with bright level and colour, such as to stop further perception of the message because of the persistence of the image on the eye retina.

Typically, with the tachistoscope, a white image is used with higher brightness than the brightness of the message, for instance a double message brightness value.

As it will be explained further, these eraser images disturb the observer's sight and therefore they are not good for therapeutic programs, in which the subliminal messages are repeatedly presented for a time of five to ten minutes.

This is an important problem, which, as it will explained in the following, has been solved by the inventor.

However, according to the inventor, this method does not achieve any result because the image carrying the message appears suddenly.

If an image appears suddenly, our mind will take some time to realise that something new has appeared and to understand what it is. Considering the uncertain or negative results obtained with the known researches, a hypothesis can be formulated that if the subliminal message appears suddenly, it is impossible to perceive it even at subliminal level.

In order to eliminate this problem, the inventor proposes to introduce, before the subliminal presentation of the image carrying the message, another step suitably created with technical modes, which will be better explained in the following, aimed at attracting the observer's attention.

During this step, of adjustable duration, the observer's attention is attracted, so that the observer perceives that something new is going to appear. If the message includes e.g. a word, the observer will perceive that this new thing is probably a word, although it cannot be clearly distinguished yet.

But this one word image, although not clearly distinguishable yet, attracts the attention of the observer of course at subliminal level, so that when the following subliminal presentation of the message begins, the observer is waiting for it.

After the presentation of the subliminal message, an eraser image follows, whose task is to stop perceiving the message due to the persistence of the image on the retina.

Duration of the previous attraction step has an upper limit which can vary according to the method used, but it must meet one requirement: it must be short enough, considering the method being used, to prevent the observer from intuiting the message that is going to be presented.

Therefore, while other scientists who have conducted most accurate researches have set their methods with the following temporal step sequence :
1. First image
2. Subliminal message
3. Eraser image
the inventor proposes the following step sequence :
1. First image
2. Attention drawing step
3. Subliminal message
4. Eraser image

In some cases, the attention drawing step may include and/or substitute partially the first image presentation step.

As already said, the attention drawing step has the task of attracting the attention of the observer to the fact that something similar to the message, or roughly similar thereto, is appearing. Consequently, when the subliminal message at last appears, it was something waited for and the observer is not confused, because his attention was already pre-activated.

Experiments conducted by the inventor, in co-operation with psychologists, have demonstrated the effectiveness achieved with the messages presented in this way, therefore the validity of the attention drawing step.

To achieve the result, the attention drawing step must have a duration that is different in relation to the method used, among the ones described in the following. During the attention drawing step, some elements forming the message must appear more and more clearly, yet avoiding perception of the message, even at subliminal level.

In other words, number, brightness and/or colour contrast with reference to the background, presentation time of those elements forming characteristic parts of the message (in most cases constituted by words) and other characteristics along with the duration of the step itself must not be sufficient for the observer to understand the message.

According to the claimed method, the attention drawing step can be performed in different modes, and before illustrating these modes, an explanation of the task of the first image is appropriate.

Using subliminal messages in psychotherapy, as proposed by the inventor, implies that a complete sentence is presented at subliminal level word after word (or possibly with subsequent short word groups).

This sentence must be displayed many times and the observer has to look at the display for a period of up to ten minutes, while the sequence including a first image, a series of images forming the attention drawing step, an image carrying the subliminal message, and the eraser image, is displayed.

In order to avoid sight overtiredness, the image sequence should be free of brightness sharp changes. Consequently, even in those cases when a first image is not necessary, and could be replaced with a dark step, it will be anyway advisable to provide a first image having a certain brightness level, just to avoid disturbing sharp brightness changes.

### DESCRIPTION OF THE PROPOSED METHODS FOR PUTTING INTO PRACTICE THE ATTRACTING ATTENTION STEP

1. Starting from a first image, during the attention drawing step, quantity and/or brightness of the graphic elements forming the image (dots, segments, parts of the image) are decreased.
   Simultaneously, graphic elements forming the message are made to appear by progressively increasing their brightness and/or number, in case not all the graphic elements forming the image carrying the message had appeared at the beginning (also colour variations can be used).
   In this way, gradual disappearance of the first image along with gradual appearance of the image carrying the message are obtained. The message will be perceived, at subliminal level, only at the end of the attention drawing step.
   Gradual disappearance or appearance of images can be made in such a way that it can be seen as a continuous or stepped transformation. For example, if the message is made to appear by presenting a few graphic elements progressively increasing, the message can be also presented with only two images in sequence: an uncompleted image and a complete image.
   When the message includes one word, also emphasised places where the letters will be displayed can be considered as elements of the message.
   Graphic elements of the message include small parts, segments, dots of the image. For example, if the image includes words, the graphic elements may include the letters forming the words, or only parts and/or segments and/or dots belonging to these letters.
   The attention drawing step can be easily made by using a LED display controlled by a microprocessor. In this way, very short image refresh time can be obtained: about one millisecond. With such short time, during the attention drawing step, images can be presented with a desired cadence.
   Since these displays generally cannot have pixels of different brightness in the same view (i.e. some pixels of certain brightness form the first image while other pixels of different brightness form the image carrying the message), the inventor suggests alternating the presentation of the first image, gradually fading, with the images carrying the message which are gradually appearing.
   Pixels are formed by the above mentioned luminous LEDs of the display.
   Considering the high refresh frequency of the display, the images that can be alternately displayed with the most convenient frequency, are perceived by the observer's eye as a unique image getting progressively transformed.
   To put the claimed method into practice using a microprocessor controlled display, it is necessary to use a memory which has stored therein all the images forming the first image while fading (e.g. the same image can be used no matter of the message that will be displayed later on). The memory will also have stored therein also the images carrying the message progressively appearing.
   To obtain this configuration, a series of images for each word, or group of words which are presented together, must be input in the memory.
   According to a simpler mode, when the message includes words, a series of images for each letter and related graphic signs is stored in the memory, these images representing in a temporal sequence the letter or the graphic sign getting progressively displayed.
   The images carrying the message will be formed by a combination of the images concerning each letter making up the word or words. This mode makes the programming of the message display easier.
   Alternating the images is not strictly necessary while using cathode ray tube screens (CRT), liquid crystal displays (LCD), plasma displays, or other displays which have a similar image scan technique.
   In fact, using these displays each image can have pixels of different luminosity.
2. Another embodiment of the proposed method differs from the previous one in that the perception of first image fades due to natural toning down of the luminous intensity persisting on an observer's eye retina.
   Thus, the presentation sequence will be as follows: first image, stop of first image presentation and start of presentation of the series of images carrying the message, which is gradually formed (as in the previous embodiment).
   The observer perceives the step formed by the first image which is faded by decreasing the luminosity, as superimposed, at least in a first time, on the message image, which is gradually appearing.
   Therefore, also in this case, there is an attention drawing step, which meets the above mentioned requirements. Like in the previous case, according to this embodiment display devices as described can be used.
3. According to a further proposed mode, a suitably prepared image, capable of masking the message, is displayed before the message image.
   The message masking image is an image, whose graphics, luminosity, and possibly colours, make it impossible, or longer in time, for the observer to perceive the message, if both the images are presented simultaneously and superimposed.
   Long in time means that the time necessary for the observer to perceive the message is longer than the duration of the attention drawing step.
   Anyway, to avoid limit situations, it is advisable to use images, which make the message perception impossible, independently from the time.
   Starting from the presentation of a masking image, which can substitute the first image, and at a certain point superimposing the message image, which is presented until the end of the step, the observer perceives an image which is the sum of the two images, but is not able to decode the message. If in this moment, the first image is weakened by decreasing its luminosity and/or removing graphic elements which form it, the attention drawing step of necessary length is obtained.
   Also this mode uses the displays described above.
   In order to avoid the necessity to prepare different masking images for each message, the inventor proposes to create one masking image for each letter of the alphabet and for each masking image a series of gradually fading images. The images thus created would be then stored in a memory unit.
   The masking image of the message will be formed by a combination of the masking images of the single letters forming the message.
   The inventor proposes another, still simpler technical feature. Instead of graphics with continuous lines, graphics including clearly separated sections and/or points are used for creating the message image as well as the masking image. In this way, it is easy to identify images, each of which masks any one of the letters of the alphabet, or anyway, any other graphic image, provided that also the latter images are created by using graphics including clearly separated sections and/or points.
   For instance, not limitative, if we use for each block letter a matrix including seven lines with five graphic elements activated in each line, which gives in all thirty five elements, we can easily compose all block letters of the alphabet by activating some of these elements, and likewise, we can easily form first images, which added to the image of any letter, mask it.
   This effect can be obtained by activating alternate lines or alternate columns, or alternate elements in each line offsetting alternate lines, i.e. beginning one line with the first element activated and the next line with the second element activated, and so on, or activating alternate elements only in two upper lines and in two lower lines of the matrix, or activating alternately diagonals of the elements, or a still different combination of these figures.
   While using higher definition displays, as well as cathode ray tube screens (CRT), where more pixels can be used to form a letter, the sections and points are obtained by activating a number of pixels necessary for forming each of these elements.
4. According to another mode, which has been examined by the inventor and which can be considered a particular and limit case of the previous mode, the fading of the brightness intensity of the masking image, independently from the kind of graphics used, is perceived by exploiting the natural toning down of the luminous intensity of the image persisting on the observer's eye retina after the image has been removed.
   At this point a more precise explanation is needed.
   Each image, which is presented directly before the message, provided that its luminosity is not lower than the message luminosity, makes it difficult to perceive immediately the message, due to the persistence on the eye retina. A very short period of time must pass, a millisecond or a little longer, so it is always a sudden appearance.
   If the image preceding the presentation of the message is a masking image, as defined before, this period of time increases.
   Using some particular technical features with respect to the luminosity level (the lower the luminosity level, the slower toning down of the image persisting on the retina), the inventor has managed to bring the above mentioned period of time to about seven milliseconds.
   Although the efficiency of the messages presented using this mode is lower than the efficiency of messages presented using other previously described modes, however an efficient feeble is obtained.
   In order to increase the efficiency, the inventor proposes to present the message for at least a rapid flash of subliminal duration, before the masking image.
   If this rapid presentation of the message is followed by the presentation of erasing image, having also masking characteristics, for a period of e.g. some dozens of milliseconds, the above described procedure can be carried out.
   As will be explained in point 6 later on, this first flash is not completely perceived even on a subliminal level, it remains marginal to the attention field, but is sufficient to attract the attention to the message. Consequently, the message will be presented as described in this mode: its efficiency is increased, because the attention is already attracted.
   This flash of the message can be presented also with other modes, described above.
   The best displays used for carrying out this mode, are displays which do not feature appreciable persistence of the luminous elements, as the LED displays.
   While using the displays featuring this persistence, as cathode ray tube screens (CRT) (with luminous persistence of phosphors), it is necessary to take this persistence into consideration and advantageously use these displays in spaces of controlled luminosity, to reduce the variation of perception time of the persisting luminosity of the phosphors connected to the environmental illumination change.
5. According to a still further mode, obtained by a variation of the third and fourth modes, the message image is presented with a luminosity, which is not constant for the whole duration of the step, but is changed to adjust the contrast with the masking image, thus obtaining still more gradual and longer attention drawing step.
6. According to yet another proposed mode for attracting attention, during the attention drawing step, rapid images of the message are presented for some milliseconds, therefore at subliminal level, i.e. the presentation length of these message images is not long enough to allow the observer to perceive them at conscious level.
   It is to be recalled that, as pointed out by the inventor, a subliminal message presented suddenly using the methods described in prior art, is difficult to be perceived even on subliminal level. The messages presented in this way remain marginal to the attention field and that is why they are weak and of uncertain effect.
   Thus, the inventor's reasoning is as follows: if we present a subliminal message once or more times one after another, and each time the subliminal message is followed by the erasing image, the attention is drawn to the fact that something is appearing, even on subliminal level, and that this thing is similar to e.g. a word, although still unclear, and in this way the very subliminal presentation, that follows, becomes perceivable, obviously on subliminal level, due to the fact that we have attracted attention to the presentation. Therefore, the sequence will be as follows:
   1. presentation of the first image,
   2. rapid presentation of the message,
   3. presentation of the erasing image,
   4. new rapid presentation of the message and relative erasing image (if this is the case),
   and so on. The attention attracting step ends with the penultimate presentation of the message and relative erasing step, while the last presentation is the very subliminal presentation.
   The length of these flashes of the messages can be different from the length of the subliminal message presentation, however, it cannot allow conscious perception of the message.
   The length of the presentation of the erasing image, between one message and another must be short enough not to distract attention from what is happening, but, on the other hand, it must be long enough to fulfil the erasing task and to avoid the perception of different flashes summed up.
   Very interesting results have been obtained by the inventor with the following sequence:
   1. first masking image,
   2. message image lasting a millisecond,
   3. erasing message lasting twenty milliseconds,
   4. presentation of subliminal message image lasting three milliseconds,
   5. erasing message.
7. A last mode obtained by the combination of the previous modes.

After the subliminal presentation step has been completed, the erasing image is presented immediately.

The inventor, contrary to other scientists, prefers not to use images of uniform luminosity much higher than the luminosity of previous images, although it is possible.

In order not to tire the observer's sight, instead of using the luminosity changes, the inventor used graphic images which are able, due to the graphics along with luminosity almost the same as the message luminosity, and possibly with the colour, to erase the image persisting on the retina, after its presentation has been finished.

According to the inventor, two conditions must be respected in order to create these images.
First: compose such an image that, if presented simultaneously with and superimposed on the message image, does not allow to recognise the message, like masking images.
Second: the image composed in this way must not show the message in reverse way, when the message is removed (turned off) after a presentation superimposed on the message.

Otherwise, as soon as the message image persisting on the retina tones down enough, its reverse image will appear.

For instance: if the message is the letter A, an image can be created that leaves the letter A or its part in reverse, so it is still possible to understand what letter it is. If we superimpose this image on the image of the letter A, respecting the dimensions, the letter cannot be distinguished any longer, however, if we remove the image of the letter, we read it again in reverse. Therefore, this image is masking, but not erasing.

Also the erasing images can be created with the same technical provisions, thus, it is possible to create erasing images for each letter of graphic sign, or to use graphics including clearly separated sections and/or points.

The images formed in this way have also the following advantage: the same image can be used as a masking and as erasing image.

The use of erasing images created by graphics including sections and/or points, along with the message created by the same graphics is not merely a matter of simplicity.

There is another very important aspect. If the whole message is formed by many words, as usually happens, and the words are presented one at a time (as many single messages), and the above described technical provision is not used, there are different masking and erasing images for each word, which distracts the observer's attention, thus negatively influencing the messages efficiency.

Therefore, the use of erasing or masking images created by graphics including clearly separated sections and/or points, together with the use of message images created by the same graphics, is an improvement of the above described method and that is why its coupling to these methods will be claimed later on.

According to the previous methods, in order not to distract the observer's attention, it is advantageous, where possible, to use the first image, which is identical with the erasing image, thus reducing image variations, when the sequences of words are presented one after another.

Independently from the chosen method, it is advisable to keep on the display an always activated area of equal dimensions, so as to avoid rapid changes of the image dimensions, which would tire the observer's sight.

If the messages formed by words are used, it is advisable to keep, for the images of superliminal presentation length, an always activated area corresponding to the words which will be presented at subliminal level, which is at least as big as the longest word of the message.

That being stated, the inventor finds it advantageous to keep also the subliminal images of almost the same dimension, adding points or other graphic signs on the right and on the left of the shortest words, to bring the dimension to the desired one.

The reason is that when the subliminal message is presented, the observer perceives a fluttering. If the dimension of this fluttering changes, the observer not only feels a light discomfort, but the observer tends to guess the message words on the grounds of their length, especially if the contents of the message has been told him for correctness's sake. This could be an obstacle in the treatment. Using this technical provision, the described methods may be more efficient.

## Claims

1. Method for communicating subliminal visual messages including presentation of a first image, presentation of a message image, and presentation of an erase image of the message image, the method being characterised in that it includes an attention attracting step between said presentation of the first image and said presentation of the message, during which attention attracting step the attention is attracted to the message image which is being presented.

2. Method as in claim 1, characterised in that the attention attracting step includes gradual reducing the quantity and/or luminance intensity of the graphic elements composing said first image and/or changing their colours, and, at the same time, beginning exposure of the graphic elements composing the message image by gradual increasing their luminance intensity, and/or quantity and/or changing their colours.

3. Method as in claim 2, characterised in that said first image luminance intensity is faded and the message image is gradually formed by alternating the presentation of the first image, which is gradually fading, with the presentation of the message image, which is gradually formed.

4. Method as in claim 1, characterised in that said attention attracting step uses the natural toning down of the luminous intensity of said first image persisting on the observer's eye retina and simultaneous beginning of exposure of graphic elements composing the message image by gradual increasing of their luminance intensity and/or quantity and/or by changing their colours.

5. Method as in claim 1, characterised in that said first image includes a presentation of a masking image.

6. Method as in claim 5, characterised in that the attention attracting step begins from a masking image, on which the message image is superimposed, and afterwards, the masking image is faded by gradually decreasing its luminance intensity and/or removing graphic elements composing said masking image and/or changing their colours, so that said message image gradually becomes perceivable.

7. Method as in claim 6, characterised in that the presentation of said first image fading and the display of the message image are obtained by alternating the presentation of the gradually fading first image with the display of the message image.

8. Method as in claim 6, characterised in that said masking image, as well as the message image, are obtained by graphics including clearly separated sections and/or points.

9. Method as in claim 8, characterised in that also said erase image of said message image is obtained by graphics including clearly separated sections and/or points.

10. Method as in claim 6, characterised in that the brightness intensity of said message image is changed, so as to adjust its contrast with the masking image which is fading down.

11. Method as in claim 6, characterised in that another rapid presentation of the message image, of subliminal duration, followed by relative erase image, is made before the presentation of the sequence of images.

12. Method as in claim 6, characterised in that the perception of said masking image is faded by exploiting the natural toning down of said luminous intensity persisting on an observer's eye retina.

13. Method as in claim 1, characterised in that said attention attracting step includes a sequence of rapid presentations of one or more message images of subliminal duration and showing the same message being subsequently displayed, each of which is followed by a relative erase image.

14. Method as in claim 1, characterised in that the said first image is identical to the said erase image.

15. Method as in claim 13, characterised in that said message is obtained by graphic elements including clearly separated sections and/or points and is associated with a relative erasing image, which is likewise obtained by graphic elements including clearly separated sections and/or points.

16. Method as in claim 13, characterised in that said message is surrounded by further points, sections or graphic elements aimed at defining clearly said message borders according to predetermined dimensions.
